# FASCICULE DE BREVET EUROPEEN

(11) **EP 1 578 289 B1**
(45) Date de publication et mention de la délivrance du brevet: **15.02.2017**
(21) Numéro de dépôt: 03815409.2
(22) Date de dépôt: 16.12.2003
(51) Int. Cl.: A61B 17/70

(54) **DISPOSITIF A PLAQUE ANTERIEURE POUR MAINTIEN DU RACHIS**
VORRICHTUNG MIT VORDERPLATTE FÜR WIRBELSÄULENABSTÜTZUNG
DEVICE COMPRISING ANTERIOR PLATE FOR VERTEBRAL COLUMN SUPPORT

(30) Priorité: 17.12.2002 FR 0216235
(43) Date de publication de la demande: 28.09.2005
(73) Titulaire: Lape Medical, 74950 Scionzier (FR)
(72) Inventeur: GRADEL, Thomas, F-74130 AYZE (FR); LEMAIRE, Jean-Philippe, F-21910 Saulon la Chapelle (FR)
(74) Mandataire: Cabinet Poncet
(86) Numéro de dépôt international: PCT/FR2003/003735
(87) Numéro de publication internationale: WO 2004/064654

(56) Documents cités:
- EP-A- 0 726 064
- EP-A- 1 093 763
- DE-U- 9 314 297
- US-A- 5 713 898
- US-A1- 2002 068 940

## Description

### DOMAINE TECHNIQUE DE L'INVENTION

La présente invention a pour objet un dispositif permettant d'assurer le maintien des vertèbres dans une position désirée. Un tel dispositif est utilisé pour le traitement d'un rachis présentant une déviation anormale, d'origine dégénérative ou traumatique.

On peut par exemple traiter des arthroses ou des fractures vertébrales, corriger des déviations de colonne vertébrale telles que la scoliose, la lordose et la cyphose.

On connaît notamment du document US-A-4 648 388 un dispositif de traitement du rachis comprenant des éléments d'ancrage dans les vertèbres, une tige de solidarisation à section circulaire et à surface extérieure lisse, et des coulisseaux de liaison pour relier des éléments d'ancrage à la tige de solidarisation. Les éléments d'ancrage sont des vis comportant trois parties principales, une première partie d'extrémité à filet hélicoïdal adapté pour une pénétration et une tenue dans l'os, une partie intermédiaire cylindrique lisse de diamètre réduit, et une seconde partie d'extrémité à filet hélicoïdal adapté pour le vissage d'un écrou de serrage. Les coulisseaux de liaison comportent une partie de serrage conformée pour entourer un tronçon de la tige de solidarisation, et une partie de liaison dépassant latéralement et percée de deux trous correspondants destinés à être traversés par la vis d'ancrage. On visse tout d'abord la vis d'ancrage dans l'os, on adapte ensuite le coulisseau de liaison sur la partie intermédiaire cylindrique de la vis d'ancrage, et on visse enfin l'écrou de serrage sur la seconde partie filetée de la vis de serrage pour plaquer le coulisseau de liaison contre une vertèbre et pour serrer simultanément le coulisseau de liaison autour de la tige de solidarisation.

Un tel dispositif est destiné à assurer le maintien du rachis selon une courbure appropriée. Il s'avère toutefois que le maintien mécanique assuré par ce dispositif n'est pas suffisant. En particulier, l'appui du coulisseau directement sur une vertèbre exclut toute possibilité de serrage efficace, par suite de la faible résistance mécanique à la compression de la vertèbre, de sorte qu'il existe un risque majeur de glissement et de rotation du coulisseau sur la tige de solidarisation. Egalement, ce dispositif est destiné à être implanté sur la face postérieure du rachis, mais n'est pas adapté à une implantation par voie antérolatérale sur la partie latérale des vertèbres. En outre, lorsque le coulisseau est en position sur la vis, il n'est plus possible d'engager latéralement la tige de solidarisation dans le coulisseau.

Le document WO 91 11967 décrit un dispositif de traitement du rachis, par voie postérieure, comprenant des vis pédiculaires à double filetage et plateau intermédiaire d'arrêt, sur lesquelles on rapporte un coulisseau conformé pour recevoir et retenir une tige de solidarisation. Le coulisseau comporte une rainure inférieure dans laquelle s'engage le plateau d'arrêt pour éviter sa rotation par rapport à la vis, et comporte une rainure supérieure dans laquelle s'engage la tige de solidarisation. La partie filetée de vis traverse le coulisseau, et un écrou de blocage à portée inférieure tronconique se visse sur la partie filetée et force latéralement la tige de solidarisation dans la rainure supérieure du coulisseau pour son blocage. La portée conique de l'écrou, en appui sur la forme cylindrique de tige de solidarisation, ne permet pas d'assurer une solidarisation suffisamment rigide de la tige de solidarisation. En outre, lorsque le coulisseau est fixé sur la vis par l'écrou, il n'est plus possible d'engager latéralement la tige de solidarisation.

Le document EP 1 093 763 A décrit un dispositif de maintien du rachis conforme au préambule de la revendication 1. Dans ce document, le coulisseau comporte quatre arêtes inférieures qui sont toutes latéralement à l'écart de la zone de réception recevant la tige de liaison. La zone de réception de tige de liaison est en partie médiane du coulisseau. Une telle disposition ne permet pas de placer la tige de liaison en position déportée vers l'arrière de la colonne vertébrale. Il en résulte une relative difficulté de pose par voie antérolatérale, et une stabilité insuffisante dans les vertèbres lors d'efforts mécaniques importants entre les vertèbres successives.

Le document US 5,713,898 A décrit un dispositif de maintien du rachis comportant deux vis osseuses destinées à s'engager dans deux trous opposés, et comportant quatre pointes proches des vis et une zone de réception centrale pour une tige transversale décalée par rapport aux pointes. Dans ce document, il n'est pas, non plus, possible de déporter la tige de liaison vers l'arrière de la colonne vertébrale.

Les documents US 2002/0068940 et DE 93 14 297 U décrivent d'autres structures pour un dispositif de maintien du rachis, comportant deux vis osseuses pour deux tiges de liaison. Les structures ne permettent pas de déporter les tiges de liaison vers l'arrière de la colonne vertébrale.

### EXPOSE DE L'INVENTION

Le problème proposé par la présente invention est de concevoir une nouvelle structure de dispositif de traitement de rachis à tige de solidarisation lisse et coulisseaux de liaison pour éléments d'ancrage, qui assure une solidarisation nettement plus efficace des vertèbres et une plus grande facilité de pose et de réglage de position des éléments les uns par rapport aux autres en tridimensionnel, et qui soit adapté à une pose par voie antérolatérale. On cherche en particulier à autoriser l'amenée et le retrait de la tige de solidarisation par déplacement latéral sur un coulisseau déjà en position sur une vis ou autre élément d'ancrage, à faciliter la fixation du coulisseau sur une vertèbre tout en améliorant sa stabilité, et à augmenter la capacité de reprise des efforts mécaniques entre les vertèbres successives.

Pour cela, le dispositif de maintien du rachis selon la présente invention comprend au moins un coulisseau de liaison pour relier une vis d'ancrage à une tige de solidarisation, le coulisseau de liaison étant une structure allongée selon une direction générale d'allongement, le coulisseau de liaison comprenant un premier trou conformé pour le passage et la fixation de la vis d'ancrage, le coulisseau de liaison comprenant des moyens de réception pour recevoir un tronçon de la tige de solidarisation orientée selon un axe transversal perpendiculaire à la direction d'allongement et pour recevoir des moyens de serrage permettant de serrer ou desserrer sélectivement la tige de solidarisation dans lesdits moyens de réception ; le coulisseau de liaison comprend deux pointes conformées pour pénétrer dans l'os d'une vertèbre et retenir ainsi le coulisseau de liaison sur la vertèbre ; les deux pointes sont disposées dans la zone de coulisseau comportant les moyens de réception, sur la face intérieure du coulisseau opposée aux moyens de réception eux-mêmes prévus sur la face extérieure du coulisseau de liaison ; les deux pointes sont parallèles et décalées l'une de l'autre dans la direction de l'axe transversal du coulisseau de liaison, parallèlement à la tige de solidarisation ; dans les moyens de réception, la tige de solidarisation est engagée en position extrême, à l'opposé de la vis d'ancrage dans la direction générale d'allongement.

Les deux pointes placées au voisinage d'une extrémité du coulisseau, et la vis d'ancrage engagée au voisinage de l'extrémité opposée du coulisseau, réalisent une fixation stable selon trois points en triangle, qui augmente la stabilité du dispositif mis en place sur le rachis par voie antérolatérale, et qui permet de positionner la tige de solidarisation au plus près vers l'arrière du rachis. Simultanément, on facilite l'accès aux vis de serrage.

Selon une réalisation pratique, chaque pointe est une structure plate généralement triangulaire dans un plan perpendiculaire à la direction de l'axe transversal. Chaque pointe peut avantageusement comporter des dents de retenue sur les deux arêtes du triangle.

De préférence, la face intérieure du coulisseau, destinée à porter contre la vertèbre, est sensiblement cylindrique concave à profil circulaire. On favorise ainsi la stabilité du coulisseau sur la vertèbre.

Pour améliorer le maintien du coulisseau sur la vertèbre, chaque pointe se développe selon une direction sensiblement radiale de la face intérieure cylindrique du coulisseau de liaison, et le premier trou présente un axe sensiblement radial par rapport à la face intérieure cylindrique du coulisseau de liaison, de sorte que la pointe et la vis d'ancrage sont convergentes vers la vertèbre.

Selon un mode de réalisation avantageux, facilitant les opérations d'assemblage des éléments du dispositif après fixation des éléments d'ancrage sur le rachis, le coulisseau comprend :
- une rainure extérieure transversale,
- une portée cylindrique formant un premier bord de la rainure transversale à l'opposé du premier trou, et conformée pour recevoir le tronçon de tige de solidarisation,
- un trou de serrage, distinct du premier trou, prévu dans le fond de la rainure transversale à l'écart du premier bord de la rainure transversale selon une distance supérieure au diamètre de la tige de solidarisation,
- une portée oblique constituant le second bord de la rainure transversale et inclinée par rapport à l'axe du trou de serrage.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue de face d'un dispositif de maintien du rachis selon un mode de réalisation de la présente invention ;
- la figure 2 est une vue du côté gauche du dispositif de la figure 1;
- la figure 3 est une vue de dessous du dispositif de la figure 1 ;
- la figure 4 est une vue en perspective du dispositif de la figure 1 ;
- la figure 5 est une autre perspective du dispositif de la figure 1 ; et
- la figure 6 illustre l'implantation du dispositif de maintien selon l'invention sur un rachis.

### DESCRIPTION DES MODES DE REALISATION PREFERES

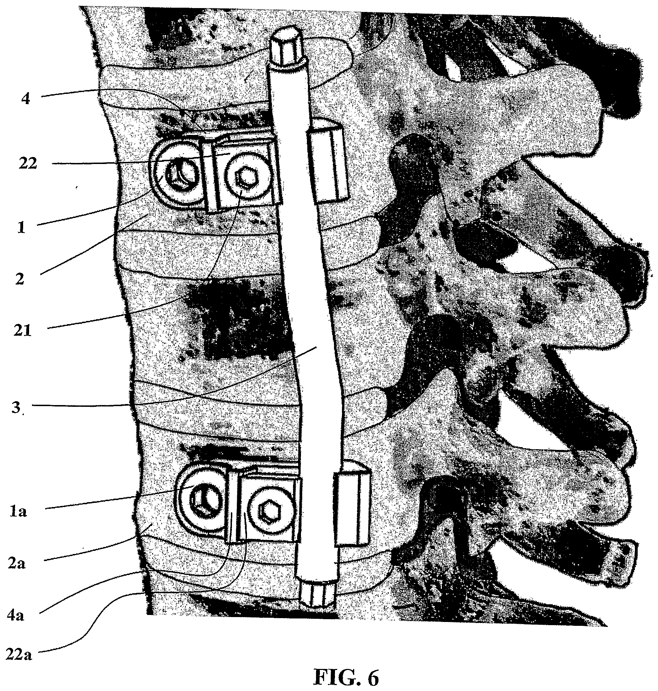

En considérant tout d'abord la figure 6, on a illustré un dispositif de maintien du rachis selon l'invention adapté à un rachis.

Le dispositif de maintien du rachis selon l'invention comprend au moins un coulisseau de liaison 4 qui relie une vis d'ancrage 1 à une tige de solidarisation 3. Le coulisseau de liaison 4 est fixé à une vertèbre 2 par la vis d'ancrage 1. De même, un second coulisseau de liaison 4a est fixé à une seconde vertèbre 2a par une seconde vis d'ancrage 1a. La tige de solidarisation 3 est fixée à l'un et l'autre des coulisseaux de liaison 4 et 4a, de sorte que le dispositif assure une liaison mécanique entre les deux vertèbres 2 et 2a.

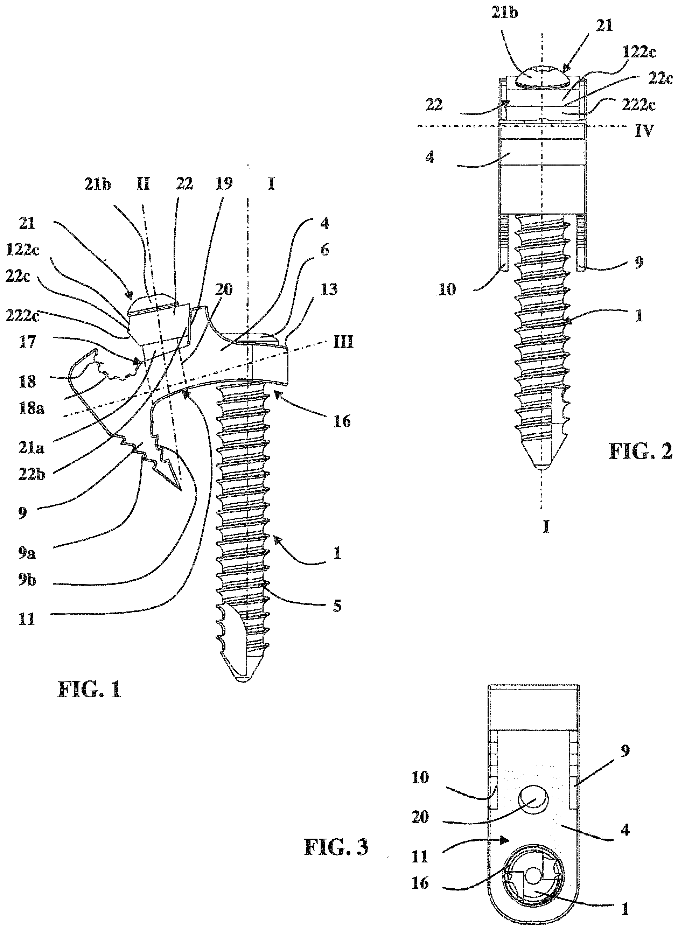

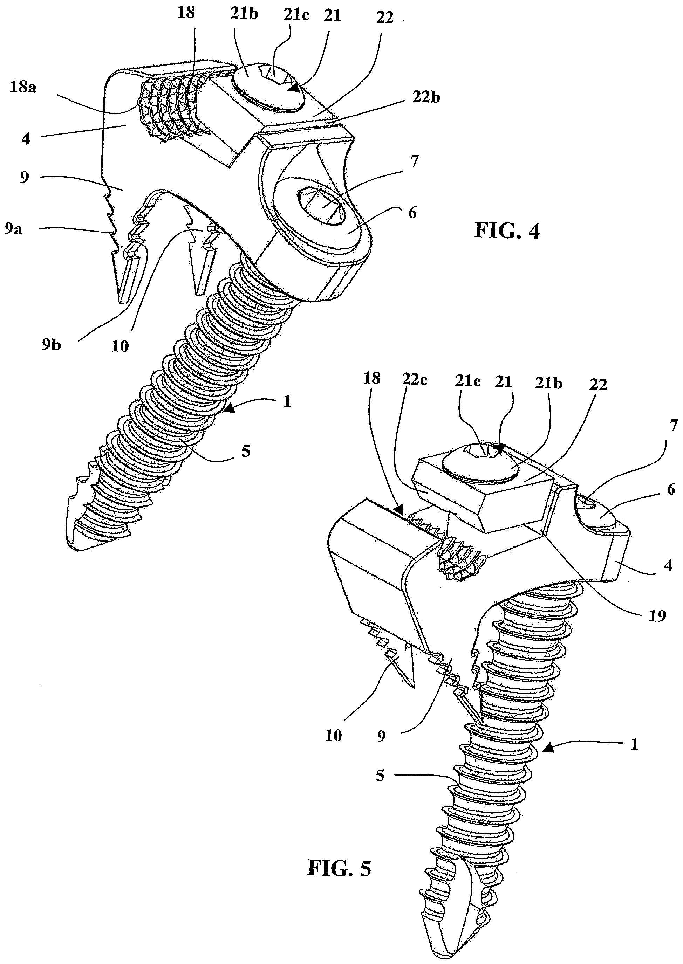

En se référant maintenant aux figures 1 à 5, on retrouve le coulisseau de liaison 4 et la vis d'ancrage 1.

La vis d'ancrage 1 comprend une tige filetée 5 conformée pour se visser dans l'os d'une vertèbre, et comprend une tête 6 à trou axial à six pans 7 pour l'introduction d'un outil de vissage.

Le coulisseau de liaison 4 est une structure allongée selon une direction générale d'allongement III, limitée par une face intérieure 11 destinée à porter contre une vertèbre, et limitée par une face extérieure 13. Le coulisseau de liaison 4 comprend un premier trou 16 conformé pour le passage de la tige 5 de la vis d'ancrage 1 selon une direction I généralement perpendiculaire aux faces intérieure 11 et extérieure 13. En se vissant dans l'os de la vertèbre, la vis d'ancrage 1 se fixe alors au coulisseau de liaison 4 par le fait que sa tête 6 vient porter sur la face extérieure 13 du coulisseau de liaison 4 autour du premier trou 16.

Le coulisseau de liaison 4 comprend des moyens de réception, par exemple une portée cylindrique 18 transversale ouverte sur la face extérieure 13, pour recevoir un tronçon de la tige de solidarisation 3, et comprend des moyens de serrage, tels qu'un cavalier 22 et une vis de serrage 21, pour serrer ou desserrer la tige de solidarisation 3 dans ladite portée cylindrique 18 transversale des moyens de réception. En position d'assemblage, telle qu'illustrée sur la figure 6, la tige de solidarisation 3 se trouve ainsi orientée selon un axe transversal IV (figure 2) perpendiculaire à la direction générale d'allongement III (figure 1) du coulisseau de liaison 4.

Selon l'invention, le coulisseau de liaison 4 comprend au moins une pointe 9 conformée pour pénétrer dans l'os d'une vertèbre et pour retenir ainsi le coulisseau de liaison 4 sur la vertèbre.

Dans la réalisation illustrée sur les figures, le coulisseau de liaison 4 comprend deux pointes 9 et 10, parallèles l'une à l'autre et décalées l'une de l'autre dans la direction de l'axe transversal IV (figure 2) du coulisseau de liaison 4, parallèlement à la tige de solidarisation. La ou les pointes 9 et 10 sont disposées dans la zone de coulisseau comportant les moyens de réception à portée cylindrique 18 transversale, sur la face intérieure 11 du coulisseau de liaison 4, en opposition avec les moyens de réception qui sont eux-mêmes prévus sur la face extérieure 13.

Chaque pointe 9 ou 10 telle qu'illustrée est une structure plate, généralement triangulaire, dans un plan perpendiculaire à la direction de l'axe transversal IV. De préférence, chaque pointe 9 et 10 comporte des dents de retenue telles que les dents 9a et 9b sur les deux arêtes du triangle.

La face intérieure 11 du coulisseau 4, ou face destinée à porter contre la vertèbre, est sensiblement cylindrique concave à profil circulaire dans le plan d'allongement du coulisseau de liaison 4 contenant les axes I et III. Le rayon de la partie cylindrique peut avantageusement être d'environ 25 à 35 millimètres, pour se conformer à la courbure anatomique de face antérolatérale d'une vertèbre.

Sur la figure 1, chaque pointe 9 et 10 se développe selon une direction sensiblement radiale de la face intérieure cylindrique 11 du coulisseau de liaison 4, et le premier trou 16 présente un axe I sensiblement radial par rapport à la face intérieure cylindrique 11 du coulisseau de liaison 4. De la sorte, les pointes 9 et 10 et la vis d'ancrage 1 convergent vers la vertèbre et s'opposent à l'arrachement. L'effet anti-arrachement est encore amélioré en prévoyant que les pointes ont une longueur égale à environ l'écart entre leur base et le premier trou 16.

Le coulisseau de liaison 4 comprend le premier trou 16 conformé pour le passage de la tige 5 de la vis d'ancrage 1, et comporte sur sa face extérieure 13 une rainure transversale 17 extérieure conformée à la fois pour recevoir le tronçon de la tige de solidarisation 3 et pour comporter des moyens de serrage permettant de serrer ou de desserrer sélectivement la tige de solidarisation 3 dans la rainure transversale 17.

La rainure transversale 17 comprend la portée cylindrique 18 qui constitue son premier bord et qui est conformée pour recevoir le tronçon de tige de solidarisation 3, par exemple en l'enveloppant sur environ 120° comme illustré sur les figures 1, 4 et 5. De préférence, la portée cylindrique 18 comporte des reliefs anti-glissement 18a qui s'opposent efficacement, après serrage, à tout déplacement de la tige de solidarisation 3 dans le coulisseau de liaison 4 tant en translation qu'en rotation.

Le second bord de la rainure transversale 17 est constitué par une portée oblique 19, inclinée de façon que la rainure transversale 17 soit évasée vers l'extérieur.

Le coulisseau de liaison 4 comporte un trou de serrage 20 taraudé, distinct du premier trou 16, et prévu dans le fond de la rainure transversale 17 du coulisseau de liaison 4. Le trou de serrage 20 est à l'écart du premier bord de la rainure transversale 17 selon une distance supérieure au diamètre de la tige de solidarisation 3, pour autoriser le passage de ladite tige de solidarisation 3.

Une vis de serrage 21, comportant une tige filetée 21a et une tête 21b, se visse dans le trou de serrage 20.

Le trou de serrage 20 est déporté vers les pointes 9 et 10 par rapport au premier trou 16 dans la direction générale d'allongement III, et son axe II est de préférence légèrement oblique par rapport à l'axe I du premier trou 16, les axes I et II formant un angle dont le sommet est dirigé vers l'intérieur c'est-à-dire à l'opposé de la rainure transversale 17. Ainsi le trou de serrage 20 est orienté obliquement par rapport à l'axe I du premier trou 16 dans le sens du rapprochement vers l'axe I du premier trou 16 lors du vissage de la vis de serrage 21.

La portée oblique 19 qui limite le second bord de la rainure transversale 17 est inclinée de façon à former, avec l'axe II du trou de serrage 20, un angle dont le sommet est également dirigé vers l'intérieur.

Un cavalier 22 est engagé en coin dans la rainure transversale 17 entre la portée oblique 19 et la tige de solidarisation 3. Le cavalier 22 est repoussé vers le fond de la rainure transversale 17 par la vis de serrage 21. Pour cela, le cavalier 22 est percé d'un trou de cavalier 22a traversé par la tige 21a de la vis de serrage 21, de sorte que la tête 21b de la vis de serrage 21 est en appui sur la face externe du cavalier 22 pour le repousser vers le fond de la rainure transversale 17.

De préférence, le cavalier 22 tourne librement autour de la vis de serrage 21, et est retenu axialement sur la tige filetée 21a de la vis de serrage 21 par une collerette de la vis de serrage 21 qui est engagée dans une rainure annulaire intérieure ovale du trou de cavalier 22a.

Le cavalier 22 comprend une face d'appui 22b qui est en appui glissant sur la portée oblique 19 du coulisseau de liaison 4, et comprend une face de poussée 22c opposée en appui sur la tige de solidarisation 3 pour assurer son blocage dans la rainure transversale 17. La face de poussée 22c (figures 1 et 2) comprend avantageusement une partie inférieure 222c, éventuellement plane, et orientée en oblique généralement vers le fond de la rainure transversale 17 pour être en appui contre une partie supérieure de la tige de solidarisation 3, et une partie supérieure 122c ouverte en oblique vers le haut pour faciliter l'engagement latéral de la tige de solidarisation 3.

Selon un mode de réalisation préféré, la tête 21b de la vis de serrage 21 comprend un trou axial 21c à contour polygonal pour l'introduction d'un outil de vissage, et la face externe de la tête 21b est en forme de calotte sphérique.

Une telle structure à coulisseau de liaison 4, cavalier 22 et vis de serrage 21 présente une hauteur particulièrement réduite, qui limite considérablement l'encombrement du dispositif et réduit en conséquence les troubles occasionnés par le positionnement du dispositif sur un rachis.

Dans le mode de réalisation préféré illustré sur les figures, dans la rainure transversale 17 la tige de solidarisation 3 est engagée en position extrême, à l'opposé de la vis d'ancrage 1 par rapport à la vis de serrage 21. De la sorte, lorsque le dispositif selon l'invention est mis en place sur un rachis, comme illustré sur la figure 6, la tige de solidarisation 3 se trouve au plus près de l'arrière du rachis, ce qui permet de la placer dans la meilleure position permettant de reprendre les efforts mécaniques entre les vertèbres successives 2 et 2a. Simultanément, on facilite l'accès aux vis de serrage telles que la vis 21, qui sont elles-mêmes positionnées plus en avant du rachis.

Le dispositif de l'invention permet l'utilisation d'une tige de solidarisation 3 ou de plusieurs éléments de tige de solidarisation, de courbures appropriées à la région vertébrale à traiter.

La mise en place des coulisseaux de liaison 4 ou 4a sur les vertèbres 2 ou 2a s'effectue aisément, puisqu'ils peuvent être chacun dans un premier temps appliqués en force sur une vertèbre 2 ou 2a en faisant pénétrer les pointes 9 et 10 dans l'os, puis solidarisés plus efficacement à la vertèbre 2 ou 2a par vissage de la vis d'ancrage 1. La tige de solidarisation 3 peut ensuite être adaptée par engagement latéral, en coulissant et pivotant librement sur le coulisseau de liaison 4 quelle que soit sa courbure, puis on fixe la tige de solidarisation 3 sur le coulisseau de liaison 4 par vissage de la vis de serrage 21.

Le principe de réduction du rachis déformé est tridimensionnel. Il faut transformer une courbe scoliotique orientée dans un plan proche du plan frontal en une courbe d'allure physiologique située dans le plan sagittal et présentant une courbure cyphotique, thoracique et lordotique lombaire normale.

On donne tout d'abord à la tige de solidarisation 3 une forme proche de la courbure physiologique normale, et on la positionne sur le patient, en fixant ses deux extrémités par des éléments d'ancrage 1, 1a, des coulisseaux de liaison 4 et 4a et des cavaliers 22, 22a correctement serrés. Les reliefs antiglissement 18a des coulisseaux de liaison 4 et 4a permettent ainsi un blocage très efficace de la tige de solidarisation 3, à la fois en translation et surtout en rotation. On positionne des éléments d'ancrage et des coulisseaux dans ou sur les autres vertèbres intermédiaires, on engage la tige de solidarisation 3 dans les coulisseaux de liaison intermédiaires, puis on verrouille la tige de solidarisation 3 par serrage des vis de serrage des coulisseaux de liaison intermédiaires.

La mise en place du dispositif selon l'invention peut s'effectuer dans un temps opératoire nettement plus court qu'avec les appareils connus antérieurement, étant donnée la facilité accrue avec laquelle le chirurgien peut présenter et assembler les éléments du dispositif les uns aux autres et au rachis lors d'une pose par voie antérolatérale. Et la stabilité du dispositif est nettement améliorée, permettant de reprendre des efforts mécaniques accrus entre les vertèbres successives, car la vis d'ancrage 1, en position antérieure, peut pénétrer dans une zone de vertèbre où l'os est généralement plus résistant, et simultanément la tige de solidarisation 3, en position postérieure, est mieux placée pour soutenir le rachis.

La présente invention n'est pas limitée aux modes de réalisation qui ont été explicitement décrits, mais elle en inclut les diverses variantes et généralisations contenues dans le domaine des revendications ci-après.

## Revendications

1. Dispositif de maintien du rachis, comprenant au moins un coulisseau de liaison (4) pour relier une vis d'ancrage (1) à une tige de solidarisation (3), le coulisseau de liaison (4) étant une structure allongée selon une direction générale d'allongement (III), le coulisseau de liaison (4) comprenant un unique premier trou (16) conformé pour le passage et la fixation d'une unique vis d'ancrage (1), le coulisseau de liaison (4) comprenant des uniques moyens de réception (18) pour recevoir un tronçon d'une unique tige de solidarisation (3) orientée selon un axe transversal (IV) perpendiculaire à la direction d'allongement (III) et pour recevoir des moyens de serrage (21, 22) permettant de serrer ou desserrer sélectivement la tige de solidarisation (3) dans lesdits uniques moyens de réception (18), **caractérisé en ce que** :
- le coulisseau de liaison (4) comprend seulement deux pointes (9, 10) conformées pour pénétrer dans l'os d'une vertèbre et retenir ainsi le coulisseau de liaison (4) sur la vertèbre,
- les deux seules pointes (9, 10) sont disposées dans la zone de coulisseau comportant les moyens de réception (18), sur la face intérieure (11) du coulisseau opposée aux moyens de réception (18) eux-mêmes prévus sur la face extérieure (13) du coulisseau de liaison (4),
- les deux seules pointes (9, 10) sont parallèles et décalées l'une de l'autre dans la direction de l'axe transversal (IV) du coulisseau de liaison (4), parallèlement à la tige de solidarisation (3),
- dans les moyens de réception (18), la tige de solidarisation (3) est engagée en position extrême, à l'opposé de la vis d'ancrage dans la direction générale d'allongement (III).

2. Dispositif selon la revendication 1, **caractérisé en ce que** chaque pointe (9, 10) est une structure plate généralement triangulaire dans un plan perpendiculaire à la direction de l'axe transversal (IV).

3. Dispositif selon la revendication 2, **caractérisé en ce que** chaque pointe (9, 10) comporte des dents de retenue (9a, 9b) sur les deux arêtes du triangle.

4. Dispositif selon l'une quelconque des revendications 1 à 3, **caractérisé en ce que** la face intérieure (11) du coulisseau (4), destinée à porter contre la vertèbre, est sensiblement cylindrique concave à profil circulaire.

5. Dispositif selon la revendication 4, **caractérisé en ce que** chaque pointe (9, 10) se développe selon une direction sensiblement radiale de la face intérieure cylindrique (11) du coulisseau de liaison (4), et le premier trou (16) présente un axe (I) sensiblement radial par rapport à la face intérieure cylindrique (11) du coulisseau de liaison (4), de sorte que les pointes (9, 10) et la vis d'ancrage (1) sont convergentes vers la vertèbre.

6. Dispositif selon l'une quelconque des revendications 1 à 5, **caractérisé en ce que** le coulisseau de liaison (4) comprend :
- une rainure extérieure transversale (17),
- une portée cylindrique (18) formant un premier bord de la rainure transversale (17) à l'opposé du premier trou (16), et conformée pour recevoir le tronçon de tige de solidarisation (3),
- un trou de serrage (20), distinct du premier trou (16), prévu dans le fond de la rainure transversale (17) à l'écart du premier bord de la rainure transversale (17) selon une distance supérieure au diamètre de la tige de solidarisation (3),
- une portée oblique (19) constituant le second bord de la rainure transversale (17) et inclinée par rapport à l'axe (II) du trou de serrage (20).

7. Dispositif selon la revendication 6, **caractérisé en ce que** les moyens de serrage comprennent :
- une vis de serrage (21) à tête (21b) et tige filetée (21a) se vissant dans le trou de serrage (20),
- un cavalier (22), engagé en coin dans la rainure transversale (17) entre la portée oblique (19) et la tige de solidarisation (3), et repoussé vers le fond de la rainure transversale (17) par la vis de serrage (21), avec une face d'appui (22b) en appui glissant sur la portion oblique (19), et avec une face de poussée (22c) opposée en appui sur la tige de solidarisation (3),

8. Dispositif selon la revendication 7, **caractérisé en ce que** le cavalier (22) est percé d'un trou de cavalier (22a) traversé par la tige (21a) de la vis de serrage (21) dont la tête (21b) est en appui sur la face externe du cavalier (22) pour le repousser vers le fond de la rainure transversale (17).

9. Dispositif selon l'une des revendications 7 ou 8, **caractérisé en ce que** la tête (21b) de la vis de serrage (21) comporte un trou axial (21c) à contour polygonal pour sa manoeuvre.

10. Dispositif selon l'une quelconque des revendications 7 à 9, **caractérisé en ce que** la face de poussée (22c) du cavalier (22) comprend une partie inférieure (222c) orientée généralement vers le fond de la rainure transversale (17) pour être en appui contre la tige de solidarisation (3), et une partie supérieure (122c) ouverte vers le haut pour faciliter l'engagement latéral de la tige de solidarisation (3).

## Patentansprüche

1. Vorrichtung zur Stützung der Wirbelsäule, umfassend wenigstens einen Verbindungsschieber (4) zum Verbinden einer Ankerschraube (1) mit einer Verbindungsstange (3), wobei der Verbindungsschieber (4) eine längliche Struktur hat, die sich in einer Längsrichtung (III) erstreckt, sowie eine einzige, erste Bohrung (16) für den Durchgriff und die Befestigung einer einzigen Ankerschraube (1) und wobei der Verbindungsschieber (4) ferner aufweist ein einziges Sitzmittel (18) für die Aufnahme einer einzigen Verbindungsstange (3), die sich auf einer Querachse (IV) erstreckt, die rechtwinklig zur Längsrichtung (III) verläuft, und für die Aufnahme von Klemmmitteln (21, 22), mit denen die Verbindungsstange (3) in dem einzigen Sitzmittel (18) festgeklemmt oder gelöst werden kann, **dadurch gekennzeichnet, dass**:
- der Verbindungsschieber (4) nur zwei Zacken (9, 10) hat, die so ausgebildet sind, dass sie in den Knochen einer Wirbelsäule eindringen können, um so den Verbindungsschieber (4) an der Wirbelsäule zu halten,
- die beiden einzigen Zacken (9, 10) in dem die Sitzmittel (18) aufweisenden Schieberabschnitt auf der Innenseite (11) des Schiebers gegenüber dem Sitzmitteln (18) angeordnet sind, welche ihrerseits auf der Außenseite (13) des Verbindungsschiebers (4) vorgesehen sind,
- die beiden einzigen Zacken (9, 10) zueinander parallel verlaufen und in Richtung der Querachse (IV) des Verbindungsschiebers (4) parallel zu der Verbindungsstange (3) gegeneinander versetzt sind,
- die Verbindungsstange (3) in die Sitzmittel (18) in ihrer äußersten Stellung eingreift, die der Ankerschraube (1) in der Längsrichtung (III) gegenüber liegt.

2. Vorrichtung nach Anspruch 1, **dadurch gekennzeichnet, dass** jede Zacke (9, 10) eine im wesentlichen dreieckige, flache Form hat und in einer zur Querachse (IV) rechtwinkligen Ebene liegt.

3. Vorrichtung nach Anspruch 2, **dadurch gekennzeichnet, dass** jede Zacke (9, 10) auf den beiden Kanten des Dreiecks Haltezähne (9a, 9b) hat.

4. Vorrichtung nach einem der Ansprüche 1 bis 3, **dadurch gekennzeichnet, dass** die Innenseite (11) des Verbindungsschiebers (4), die dazu dient, an der Wirbelsäule anzuliegen, ein im wesentlichen kreiszylindrisches, konkaves Profil hat.

5. Vorrichtung nach Anspruch 4, **dadurch gekennzeichnet, dass** sich jede Zacke (9, 10) von der zylindrischen Innenseite (11) des Verbindungsschiebers (4) in einer im wesentlichen radialen Richtung erstreckt und dass die erste Bohrung (16) eine zur zylindrischen Innenseite (11) des Verbindungsschiebers (4) im wesentlichen radiale Achse (I) hat, so dass die Zacken (9, 10) und die Ankerschraube (1) in Richtung auf die Wirbelsäule konvergieren.

6. Vorrichtung nach einem der Ansprüche 1 bis 5, **dadurch gekennzeichnet, dass** der Verbindungsschieber (4) umfasst:
- einen in Querrichtung verlaufenden äußeren Einschnitt (17),
- eine zylindrische Anschlagfläche (18), die einen ersten, der ersten Bohrung (16) gegenüberliegenden Rand des querliegenden Einschnitts (17) bildet und so ausgebildet ist, dass sie ein Teilstück der Verbindungsstange (3) aufnimmt,
- eine von der ersten Bohrung (16) getrennte Klemmbohrung (20) im Boden des querliegenden Einschnitts (17), der zum ersten Rand des Einschnitts (17) einen Abstand hat, der größer als der Durchmesser der Verbindungsstange (3) ist,
- eine schräge Anschlagfläche (19), die den zweiten Rand des querliegenden Einschnitts (17) bildet und zur Achse (II) der Klemmbohrung (20) geneigt ist.

7. Vorrichtung nach Anspruch 6, **dadurch gekennzeichnet, dass** die Klemmmittel umfassen:
- eine Klemmschraube (21) mit Kopf (21b) und Gewindeschaft (21a), der in die Klemmbohrung (20) eingeschraubt ist,
- einen Gleitstein (22), der keilartig in den querliegenden Einschnitt (17) zwischen der schrägen Anschlagfläche (19) und der Verbindungsstange (3) eingreift und über die Klemmschraube (21) gegen den Boden des querliegenden Einschnitts (17) gedrückt wird, wobei eine Anschlagfläche (22b) des Gleitsteins (22) in Gleitberührung an der schrägen Anschlagfläche (19) anliegt und eine gegenüberliegende Anschlagfläche (22c) an der Verbindungsstange (3) anliegt.

8. Vorrichtung nach Anspruch 7, **dadurch gekennzeichnet, dass** in den Gleitstein (22) eine Steckbohrung (22a) eingearbeitet ist, durch die der Schaft (21a) der Klemmschraube (21) greift, deren Kopf (21b) an der Außenseite des Gleitsteins (22) anliegt, um diesen gegen den Boden des querliegenden Einschnitts (17) zu drücken.

9. Vorrichtung nach Anspruch 7 oder 8, **dadurch gekennzeichnet, dass** der Kopf (21b) der Klemmschraube (21) zu ihrer Verstellung eine axiale Ausnehmung (21c) mit polygonalem Innenprofil hat.

10. Vorrichtung nach einem der Anspruch 7 bis 9, **dadurch gekennzeichnet, dass** die Anschlagfläche (22c) des Gleitsteins (22) einen unteren Abschnitt (222c) hat, der im wesentlichen gegen den Boden des querliegenden Einschnitts (17) gerichtet ist, um zum Anschlag gegen die Verbindungsstange (3) zu kommen, sowie einen oberen Abschnitt (122c), der sich nach oben öffnet, um den seitlichen Eingriff der Verbindungsstange (3) zu erleichtern.

## Claims

1. Device for vertebral column support, comprising at least one connecting sliding piece (4) for connecting an anchoring screw (1) to a fastening rod (3), the structure of the connecting sliding piece (4) being elongate in a general lengthwise direction (III), the connecting sliding piece (4) comprising a single first hole (16) conformed for the passage and fixing of a single anchoring screw (1), the connecting sliding piece (4) comprising unique receiving means (18) adapted to receive a portion of a single fastening rod (3) oriented along a transverse axis (IV) perpendicular to the lengthwise direction (III) and to receive clamping means (21, 22) for selectively clamping the fastening rod (3) in said unique receiving means (18) or releasing it therefrom, **characterized in that**:
- the connecting sliding piece (4) comprises only two points (9, 10) conformed to penetrate into the bone of a vertebra to retain the connecting sliding piece (4) on the vertebra,
- the only two points (9, 10) are disposed in the region of the sliding piece including the receiving means (18), on the interior face (11) of the sliding piece and opposite the receiving means (18), which are on the exterior face (13) of the connecting sliding piece (4),
- the only two points (9, 10) are parallel to and offset from each other in the direction of the transverse axis (IV) of the connecting sliding piece (4), parallel to the fastening rod (3),
- the fastening rod (3) is engaged in the end of the receiving means (18) opposite the anchoring screw in the general lengthwise direction (III).

2. Device according to claim 1, **characterized in that** each point (9, 10) is a generally triangular flat structure in a plane perpendicular to the direction of the transverse axis (IV).

3. Device according to claim 2, **characterized in that** each point (9, 10) comprises retaining teeth (9a, 9b) on the two sides of the triangle.

4. Device according to any one of claims 1 to 3, **characterized in that** the interior face (11) of the sliding connecting piece (4) adapted to bear against the vertebra is concave and substantially cylindrical with a circular profile.

5. Device according to claim 4, **characterized in that** each point (9, 10) extends in a substantially radial direction of the cylindrical interior face (11) of the connecting sliding piece (4) and the first hole (16) has an axis (I) that is substantially radial relative to the cylindrical interior face (11) of the connecting sliding piece (4) so that the points (9, 10) and the anchoring screw (1) converge toward the vertebra.

6. Device according to any one of claims 1 to 5, **characterized in that** the connecting sliding piece (4) comprises:
- a transverse exterior groove (17),
- a cylindrical bearing surface (18) forming a first edge of the transverse groove (17) opposite the first hole (16) and conformed to receive a portion of the fastening rod (3),
- a clamping hole (20) separate from the first hole (16) in the bottom of the transverse groove (17) separated from the first edge of the transverse groove (17) by a distance greater than the diameter of the fastening rod (3),
- an oblique bearing surface (19) constituting the second edge of the transverse groove (17) and inclined to the axis (II) of the clamping hole (20).

7. Device according to claim 6, **characterized in that** the clamping means comprise:
- a tightening screw (21) with a head (21b) and a threaded shank (21a) adapted to be screwed into the clamping hole (20),
- a jumper (22) adapted to be engaged in the manner of a wedge in the transverse groove (17) between the oblique bearing surface (19) and the fastening rod (3) and adapted to be pushed toward the bottom of the transverse groove (17) by the tightening screw (21) with a bearing face (22b) in sliding bearing engagement with the oblique portion (19) and with an opposite thrust face (22c) bearing on the fastening rod (3).

8. Device according to claim 7, **characterized in that** the jumper (22) is pierced by a jumper hole (22a) through which is passed the shank (21a) of the tightening screw (21) whose head (21b) bears on the external face of the jumper (22) to push it toward the bottom of the transverse groove (17).

9. Device according to either claim 7 or claim 8, **characterized in that** the head (21b) of the tightening screw (21) includes a polygonal contour axial hole (21c) for turning it.

10. Device according to any one of claims 7 to 9, **characterized in that** the thrust face (22c) of the jumper (22) has a lower portion (222c) oriented generally toward the bottom of the transverse groove (17) to bear against the fastening rod (3) and an upper portion (122c) open upward to facilitate lateral engagement of the fastening rod (3).
